# EUROPÄISCHE PATENTSCHRIFT

(11) **EP 3 216 425 B1**
(45) Veröffentlichungstag und Bekanntmachung des Hinweises auf die Patenterteilung: **25.12.2019**
(21) Anmeldenummer: 16159418.9
(22) Anmeldetag: 09.03.2016
(51) Int. Cl.: A61F 2/38, A61F 2/30

(54) **GELENKAUFBAU**
JOINTED STRUCTURE
ARTICULATION

(43) Veröffentlichungstag der Anmeldung: 13.09.2017
(73) Patentinhaber: WALDEMAR LINK GmbH & Co. KG, 22339 Hamburg (DE)
(72) Erfinder: Link, Helmut D., 22397 Hamburg (DE); Ewald, Jörg, 22089 Hamburg (DE); Jendro, Günther, 24568 Kaltenkirchen (DE); Dänike, Andreas, 24558 Henstedt-Ulzburg (DE); Borchers, Udo, 22850 Norderstedt (DE)
(74) Vertreter: Hoffmann Eitle

(56) Entgegenhaltungen:
- WO-A1-2006/087582
- WO-A1-2013/144392
- ES-A2- 2 429 390
- FR-A1- 2 935 894
- US-A1- 2014 025 172

## Beschreibung

### GEBIET DER ERFINDUNG

Die vorliegende Erfindung betrifft einen Gelenkaufbau für eine ein Scharniergelenk ausbildende Gelenkendoprothese sowie ein Verfahren zu dessen Verriegelung.

### STAND DER TECHNIK

Es kann notwendig oder von Vorteil sein, ein Gelenk im Rahmen einer Behandlung ruhig zu stellen. Indikationen hierfür, die eine solche Ruhigstellung eines Gelenks erfordern, sind zum Beispiel Entzündungen im Bereich des Gelenks, eine Schwächung oder Reizung des Bänderapparats, der das Gelenk umgibt, oder eine Fraktur des Knochengewebes.

Eine Ruhigstellung eines Gelenks kann dabei auch im Zusammenhang mit einem künstlichen Gelenk von Vorteil sein, das bereits implantiert oder noch zu implantieren ist. Insbesondere bei einer Entzündung im Gelenkbereich, die beispielsweise durch ein abgenutztes natives Gelenk hervorgerufen werden kann, stellt sich zudem das Problem, dass entzündetes Gewebe entfernt werden muss, jedoch noch kein Gelenkersatz implantiert werden sollte, da dessen Beweglichkeit eine weitere Reizung des umliegenden Gewebes zur Folge hat.

Aus diesem Grund wird anstelle eines vollständigen Gelenkersatzes beispielsweise zumindest zeitweise ein Arthrodesenagel implantiert, der das betroffene Gelenk blockiert, indem er es überbrückt. Sobald sich die gesundheitliche Situation des Patienten ausreichend gebessert hat, kann der Arthrodesenagel in einem erneuten Eingriff wieder entfernt werden und durch einen Gelenkersatz ersetzt werden. Hierfür muss der Arthrodesenagel wieder entfernt werden, was eine weitere Belastung des Patienten zur Folge hat. Insgesamt sind folglich zwei größere Eingriffe notwendig, um den Patienten zu behandeln.

Ist bereits ein künstlicher Gelenkersatz implantiert, so kann eine Entzündung des umliegenden Knochen- oder Weichteilgewebes oder Abnutzung eine Revision dieses Gelenks notwendig machen. Die Revision eines Gelenks ist ebenfalls ein relativ starker Eingriff, der das Gewebe eines Patienten stark belastet und häufig mit einem erheblichen Verlust an Knochengewebe verbunden ist. Deswegen ist es unter Umständen von Vorteil, auch hier das Gelenk mittels eines Arthrodesenagels ruhig zu stellen. Die Folge sind wiederum die oben bereits beschriebenen zwei Eingriffe.

Weiterhin kann nach einer Implantation eines künstlichen Gelenks oder nach dessen Revision zudem eine anfängliche Ruhigstellung des neuen künstlichen Gelenks die Heilung begünstigen, da sich der geschwächte Bänderapparat und das Knochengewebe von dem Eingriff erholen können. Auch kann es direkt nach einer Implantation eines künstlichen Gelenks zu kleineren Reizungen und Infekten kommen, die keine Revision notwendig machen, jedoch durch eine temporäre Ruhigstellung besser behandelt werden können. Hierfür gibt es derzeit nach Kenntnis der Erfinder keine praktikable Lösung, außer dem Patienten Bettruhe zu verordnen und damit dessen Bewegungsfreiheit entsprechend stark einzuschränken.

Weiterer Stand der Technik ist in WO 203/144392 A1 und WO 2006/087582 offenbart.

Insbesondere die WO2013/144392 A1 zeigt einen Gelenkaufbau für ein Kniegelenk mit einer Gelenkaufnahme und einem Gelenkkörper, die mittels eines quer zur Gelenkachse eingesetzten Verriegelunselementes gegeneinander verriegelt werden.

### ZUSAMMENFASSUNG DER ERFINDUNG

Hiervon ausgehend war es eine Aufgabe der vorliegenden Erfindung, eine Möglichkeit bereitzustellen, welche eine möglichst schonende Ruhigstellung des Gelenkbereichs ermöglicht, ohne die Mobilität eines Patienten zu stark einschränken zu müssen.

Die hierfür gefundene Lösung wird durch den Gelenkaufbau nach Anspruch 1 und die Verriegelung eines Gelenkaufbaus nach Anspruch 12 definiert. Die jeweiligen abhängigen Ansprüche definieren dabei bevorzugte Ausführungsformen des Gelenkaufbaus bzw. des Verriegelungsverfahrens.

Der Gelenkaufbau der vorliegenden Erfindung für eine ein Scharniergelenk ausbildende Gelenkendoprothese weist dabei eine Gelenkaufnahme mit einer Gelenkaufnahmefläche und einer Aufnahmeverriegelungsstruktur sowie einen Gelenkkörper auf, der in der Gelenkaufnahme aufnehmbar ist und wiederum eine Gelenkkörperfläche und eine Körperverriegelungsstruktur aufweist. Die Gelenkaufnahmefläche und die Gelenkkörperfläche sind relativ zueinander in eine Stellung bewegbar, in der die Aufnahmeverriegelungsstruktur und die Körperverriegelungsstruktur eine Verriegelungsposition einnehmen, in der sie für einen verriegelten Zustand zwischen der Gelenkaufnahme und dem Gelenkkörper in Eingriff bringbar sind.

Durch diesen Gelenkaufbau kann die Gelenkendoprothese ruhiggestellt werden und hat im verriegelten Zustand dementsprechend die Wirkung eines Arthrodesenagels. Damit wird der Drehung des Scharniergelenks vorgebeugt. Zudem kann durch ein Lösen der Verriegelung diese Blockade des Gelenkaufbaus und damit der Gelenkendoprothese wieder rückgängig gemacht werden. Als Ergebnis wird eine Reizung oder Entzündung des betroffenen Gelenks behandelt, ohne dass eine Revision der Gelenkendoprothese notwendig ist.

Weiterhin hat der Gelenkaufbau insbesondere den Vorteil, dass eine Verriegelung des Scharniergelenks durch einen kleinen Einschnitt in das Gewebe des Patienten, der die Körperverriegelungsstruktur und die Aufnahmeverriegelungsstruktur zugänglich macht, möglich ist. Ein solcher minimalinvasiver Eingriff minimiert die Belastung des Patienten.

Durch die Blockade des Gelenks wird zudem Schmerzen vorgebeugt, die anderweitig durch die normale Beweglichkeit des Gelenks hervorgerufen werden könnten. Damit ist ein Patient in der Lage, kurze Distanzen weiter selbst zurücklegen, ohne dabei Hilfe von außen zu benötigen.

Bei dem Scharniergelenk bildet der Gelenkkörper die Achse des Scharniergelenks und die Gelenkaufnahme das Lager für diese Achse aus. Derartige Scharniergelenke treten bei Tieren und bei Menschen beispielsweise beim Kniegelenk oder beim Ellenbogengelenk auf. Dementsprechend ist die Verwendung des Begriffs Patienten nicht beschränkend auf Menschen auszulegen.

Bei einer bevorzugten Ausführungsform grenzt die Aufnahmeverriegelungsstruktur an die Gelenkaufnahmefläche und die Körperverriegelungsstruktur an die Gelenkkörperfläche an.

Eine derartige Anordnung der Aufnahmeverriegelungsstruktur und der Körperverriegelungsstruktur hat den Vorteil, dass die Verriegelung in unmittelbarer Nähe der Lauffläche des Scharniergelenks erfolgt und somit ein besonders geringes Spiel im verriegelten Zustand erreicht wird, da die Verriegelung dort stattfindet, wo das Gelenk über die Laufflächen seine Beweglichkeit erhält. Folglich wird der Bewegung des Gelenks genau dort vorgebeugt, wo diese entsteht. Durch das hierdurch ermöglichte geringere Spiel wird zudem einem möglichen Versagen der Verriegelung vorgebeugt.

Bei einer weiteren besonders bevorzugten Ausführungsform ist die Aufnahmeverriegelungsstruktur als Aussparung in der Gelenkaufnahmefläche und/oder die Körperverriegelungsstruktur als Aussparung in der Gelenkkörperfläche ausgebildet.

Auch bei dieser Ausführungsform wird eine Verriegelung an den Laufflächen des Gelenks mit wenig Spiel erreicht.

Sind die Körperverriegelungsstruktur und die Aufnahmeverriegelungsstruktur komplementär zueinander ausgebildet, erfolgt eine Verriegelung durch einen direkten Formschluss zwischen den beiden Verriegelungsstrukturen.

Erfindungsgemäss sind die Aufnahmeverriegelungsstruktur und die Körperverriegelungsstruktur mit einem Verriegelungselement in Eingriff bringbar, wobei das Verriegelungselement vorzugsweise in der Verriegelungsposition in die Aufnahmeverriegelungsstruktur und die Körperverriegelungsstruktur einführbar ist.

Folglich werden bei dieser Ausführungsform die Aufnahmeverriegelungsstruktur und die Körperverriegelungsstruktur indirekt miteinander in Eingriff gebracht.

Das Verriegelungselement hat den Vorteil, dass es nicht in dem Gelenkaufbau verbleibt, wenn keine Verriegelung des Gelenkaufbaus gewünscht ist. Mit anderen Worten handelt es sich um ein getrenntes Verriegelungselement. Damit wird zum einen die Konstruktion des Gelenkaufbaus vereinfacht und zum anderen der Möglichkeit vorgebeugt, dass das Verriegelungselement das Gelenk unbeabsichtigt verriegelt. In dieser Ausführung ist das Verriegelungselement somit nicht nur lösbar sondern auch entfernbar ausgeführt. Auch hier reicht der zuvor bereits beschriebene kleine Einschnitt in Gelenknähe aus, um die Verriegelung auszuführen und wieder rückgängig zu machen.

Weiterhin ist bei dieser Ausführungsform vorzugsweise sowohl die Aufnahmeverriegelungsstruktur als auch die Körperverriegelungsstruktur als Aussparung ausgebildet. Die beiden Aussparungen bilden in der Verriegelungsposition zusammen eine Verriegelungsaussparung aus. Mit anderen Worten fluchten die Aufnahmeverriegelungsstruktur und die Körperverriegelungsstruktur in der Verriegelungsposition miteinander, sodass ein Verriegelungselement einführbar ist. Die durch die Aufnahmeverriegelungsstruktur und die Körperverriegelungsstruktur ausgebildete Verriegelungsaussparung ist bevorzugt rotationssymmetrisch, noch bevorzugter zylindrisch und am bevorzugtesten kreisförmig zylindrisch. Weiterhin erstrecken sich die Aussparungen vorzugsweise in die jeweilige Fläche, sodass ein Verriegelungselement von außerhalb der Fläche eingeführt werden kann.

Erfindungsgemäss sind die Gelenkaufnahme und der Gelenkkörper relativ zueinander um eine Rotationsachse schwenkbar und ist das Verriegelungselement im Wesentlichen parallel zu der Rotationsachse einführbar.

So wird durch die Einführbarkeit des Verriegelungselements parallel zu der Rotationsachse des Scharniergelenks ein besonders einfacher Zugang zum Gelenk von lateral oder medial für eine Verriegelung oder Entriegelung des Gelenks von außen ermöglicht.

Bei einer weiteren bevorzugten Ausführungsform weist der Gelenkkörper einen Sicherungsabschnitt für eine Sicherung des Gelenkkörpers an einer ersten Gelenkkomponente auf, wobei der Sicherungsabschnitt vorzugsweise als mindestens ein Flansch an einem Ende des Gelenkkörpers ausgebildet ist.

Der Sicherungsabschnitt verhindert eine Trennung des Gelenkkörpers und der Gelenkaufnahme nach einer Montage und gewährleistet so eine einwandfreie Funktion des Gelenkaufbaus. Zudem kann der Sicherungsabschnitt so ausgeführt sein, dass er als Anschlag dient, der ein definiertes Einschieben bei der Montage des Gelenkaufbaus ermöglicht.

Der Sicherungsabschnitt ist vorzugsweise als Flansch ausgeführt, der in Richtung der Rotationsachse an zumindest einem Ende des Gelenkkörpers angebracht ist. Vorzugsweise ist der Flansch einstückig mit dem Flansch verbunden. Eine Sicherung des Sicherungsabschnitts und damit des Gelenkkörpers kann folglich durch ein Anbringen des Sicherungsabschnitts an einer ersten Gelenkkomponente erfolgen.

Es ist möglich, an dem Gelenkkörper in axialer Richtung beidseitig Flansche vorzusehen, die im montierten Zustand beidseitig von der Gelenkaufnahme einer Trennung des Gelenkkörpers von der Gelenkaufnahme vorbeugen. Dabei kann der Gelenkkörper im unverriegelten Zustand durch die beiden Flansche nur in axialer Richtung gesichert sein und sich um die Rotationsachse des Scharniergelenks und relativ zu der ersten Gelenkkomponente drehen.

Bei einer besonders bevorzugten Ausführungsform ist der Sicherungsabschnitt mit einem Befestigungselement an der ersten Gelenkkomponente befestigt.

Folglich erfolgt über mindestens ein Befestigungselement eine vollständige Sicherung des Gelenkkörpers an der ersten Gelenkkomponente, d.h. diese ist gegen ein axiales Herausrutschen sowie eine Drehung relativ zu der ersten Gelenkkomponente gesichert. Dementsprechend kann der Sicherungsabschnitt bei dieser Ausführungsform durch lediglich einen Flansch ausgebildet sein, was eine kompakte Bauweise des Gelenkaufbaus möglich macht.

Die vollständige Sicherung über das Befestigungselement erfolgt dabei vorzugsweise außerhalb der Gelenkachse und kann am einfachsten über eine Gewindeverbindung ausgeführt werden.

Bei einer weiteren besonders bevorzugten Ausführungsform ist mindestens ein Adapterelement zwischen dem Gelenkkörper und der ersten Gelenkkomponente vorgesehen, das eine Stützfläche zum Unterstützen der Gelenkkörperfläche aufweist.

Die Verwendung eines Adapterelements zwischen dem Gelenkkörper und der ersten Gelenkkomponente ermöglicht das Nachrüsten einer bereits implantierten Gelenkendoprothese mit dem Gelenkaufbau. Dies hat den Vorteil, dass eine bereits implantierte Komponente der Gelenkendoprothese nicht aus dem Knochen entfernt werden muss und so den Patienten schont. Damit bildet der Gelenkaufbau eine sinnvolle Ergänzung für bestehende Gelenkimplantatsysteme und erweitert die Einsatzmöglichkeiten dieser.

Im verriegelten Zustand wird bei einem mit einem Adapter versehenen Gelenkaufbau vorzugsweise einer relativen Drehung zwischen der Gelenkaufnahme und dem Adapter vorgebeugt.

Bei einer weiteren Ausführungsform weist das Adapterelement eine Arretierung auf, die einer Drehung zwischen dem Adapterelement und der ersten Gelenkkomponente vorbeugt.

Diese Arretierung beugt einer Drehung zwischen dem Adapterelement und der ersten Gelenkkomponente sowohl im unverriegelten Zustand als auch im verriegelten Zustand vor. Vorzugsweise ist die Arretierung in Form von zwei Anschlagflächen ausgebildet, die im montierten Zustand mit zwei Anschlagflächen der ersten Gelenkkomponente in Drehrichtung zusammenwirken. Folglich sorgt die Arretierung dafür, dass eine Verriegelung durchgehend zwischen der Gelenkaufnahme und der ersten Gelenkkomponente erfolgt.

Über die Arretierung kann zudem über ihre entsprechende Anordnung der Verriegelungswinkel zwischen der Gelenkaufnahme und der ersten Gelenkkomponente gewählt werden. Beispielsweise kann es für einen Patienten von Vorteil sein, dass die Verriegelung im Falle eines Kniegelenks in einer Stellung des Gelenkaufbaus erfolgt, in welcher der Patient sitzt. Dies trifft insbesondere auf Patienten zu, die einen Rollstuhl verwenden.

Bei einer weiteren besonders bevorzugten Ausführungsform ist das Verriegelungselement vorzugweise über einen Gewindeabschnitt relativ zu dem Gelenkkörper fixierbar.

Über einen derartigen Gewindeabschnitt kann das Verriegelungselement relativ zu dem Gelenkaufbau gesichert werden, wenn es für eine Verriegelung des Scharniergelenks im eingeführten Zustand ist. Damit wird einem versehentlichen oder unbeabsichtigten Herausbewegen des Verriegelungselements vorgebeugt. Insbesondere bei einem Einführen des Verriegelungselements parallel zu der Rotationsachse des Scharniergelenks kann die Fixierung über den Gewindeabschnitt durch einfaches Einschrauben des Verriegelungselements in eine Gelenkkomponente, d. h. die Gelenkseite der Gelenkaufnahme oder des Gelenkkörpers, ein Adapterelement und/oder einen Sicherungsabschnitt erfolgen.

Bei einer weiteren besonders bevorzugten Ausführungsform weist die Gelenkaufnahme einen Verbindungsabschnitt für eine Verbindung mit einer zweiten Gelenkkomponente auf.

Bei dieser Ausführung wird der gleiche Vorteil erreicht, wie im Falle des oben beschriebenen Adapterelements. Genauer gesagt ist es bei dieser Ausführungsform möglich, den Gelenkaufbau bei einer zweiten Gelenkkomponente, die der ersten Gelenkkomponente funktionell gegenüberliegt, nachzurüsten. Auch hier kann somit auf eine Revision einer bereits implantierten Gelenkkomponente verzichtet werden.

Bei einer weiteren besonders bevorzugten Ausführungsform weist der Gelenkaufbau eine weitere Aufnahmeverriegelungsstruktur und/oder eine weitere Körperverriegelungsstruktur auf, die angeordnet ist, um mindestens eine weitere Verriegelungsposition für eine Verriegelung des Gelenkaufbaus bereitzustellen.

Bei dieser Ausführungsform ist es möglich, die Verriegelung des Gelenkaufbaus und damit des Gelenkersatzes in unterschiedlichen Winkelstellungen vorzunehmen. Folglich kann der Verriegelungswinkel an die jeweiligen Bedürfnisse des Patienten angepasst werden. So ist es beispielsweise nicht immer von Vorteil eine Verriegelung vorzunehmen, sodass das betreffende Körperglied in einem gestreckten Zustand ist, wie zum Beispiel bei der oben erwähnten Verwendung eines Rollstuhls.

Besonders bevorzugt werden dabei zwei, drei oder vier Aufnahmeverriegelungs- oder Körperverriegelungsstrukturen vorgesehen, die mit nur einer der jeweils anderen Struktur in eine entsprechende Anzahl von Verriegelungspositionen bringbar sind.

Weiterhin stellt die vorliegende Erfindung ein Verfahren zur Verriegelung eines Gelenkaufbaus, wie in Anspruch 1, für eine ein Scharniergelenk ausbildende Gelenkendoprothese bereit, wobei das Verfahren die folgenden Schritte umfasst. Bei einem Schritt erfolgt das Einführen eines Gelenkkörpers, der eine Gelenkkörperfläche und eine Körperverriegelungsstruktur aufweist, in eine Gelenkaufnahme, die eine Gelenkaufnahmefläche und eine Aufnahmeverriegelungsstruktur aufweist. Weiterhin sieht das Verfahren ein Bewegen der Gelenkkörperfläche und der Gelenkaufnahmefläche relativ zueinander vor, sodass die Körperverriegelungsstruktur und die Aufnahmeverriegelungsstruktur eine Verriegelungsposition einnehmen. Als Teil des Verfahrens erfolgt das Verriegeln des Gelenkkörpers und der Gelenkaufnahme in der Verriegelungsposition durch in Eingriff bringen der Körperverriegelungsstruktur und der Aufnahmeverriegelungsstruktur.

Damit ermöglicht dieser Aufbau eine Verriegelung einer Gelenkendoprothese und macht somit die weit aufwändigere und für den Patienten stärker belastende Verwendung eines Arthrodesenagels überflüssig. Zudem ist die Verriegelung vorzugsweise lösbar vorgesehen, sodass nach Erreichen des Behandlungsziels, welches durch die Verriegelung bezweckt wird, die Verriegelung wieder rückgängig gemacht werden und der Patient die Gelenkendoprothese wieder als solche verwenden kann.

Bei einer weiteren Ausführungsform des Verfahrens zur Montage eines Gelenkaufbaus erfolgt die Verriegelung zwischen der Körperverriegelungsstruktur und der Aufnahmeverriegelungsstruktur mit einem Verriegelungselement.

Der Einsatz eines Verriegelungselements zum Verriegeln des Gelenkaufbaus hat insbesondere den oben beschriebenen Vorteil, dass für die Verriegelung lediglich ein minimalinvasiver Eingriff erforderlich ist. Wenn das Verriegelungselement zudem entfernbar ausgebildet ist, ermöglicht dies eine vollständige Entfernung desselben, wenn keine Verriegelung des Gelenkaufbaus mehr notwendig ist. Dabei erfolgt auch der Eingriff zum Entfernen des Verriegelungselements minimalinvasiv.

Bei einer weiteren Ausführungsform des Verfahrens zur Montage eines Gelenkaufbaus wird der Gelenkkörper über ein Adapterelement mit einer ersten Gelenkkomponente verbunden.

Ein derartiger modularer Aufbau des Gelenkaufbaus in Bezug auf eine erste Gelenkkomponente durch die Verwendung eines Adapterelements ermöglicht den oben bereits beschriebenen vorteilhaften Einsatz bei einer bereits implantierten ersten Gelenkkomponente. Zudem ist bei Verwendung einer lösbaren bzw. entfernbaren Verriegelung keine Entnahme des Gelenkaufbaus erforderlich, was ebenfalls die Belastung eines Patienten verringert.

Bei einer besonders bevorzugten Ausführungsform weist das Adapterelement eine Stützfläche, die mit der Gelenkaufnahmefläche der Gelenkaufnahme in Kontakt gebracht wird, und eine Arretierung auf, die einer relativen Drehung zwischen dem Adapterelement und der ersten Gelenkkomponente vorbeugt.
Folglich übernimmt das Adapterelement die Lagerung des Gelenkkörpers, während über die Arretierung einer relativen Drehung zwischen dem Adapterelement und der ersten Gelenkkomponente vorgebeugt wird und damit jegliche Drehung zwischen dem Gelenkkörper und der Gelenkaufnahme direkt auf die erste Gelenkkomponente übertragen wird.

Bei einer weiteren Ausführungsform wird die Gelenkaufnahme an einer zweiten Gelenkkomponente befestigt.

Wie oben bereits beschrieben kann hierdurch eine medizinisch nicht notwendige Revision der zweiten Gelenkkomponente durch einfaches Nachrüsten des Gelenkaufbaus verhindert werden.

### KURZE BESCHREIBUNG DER FIGUREN

In den folgenden Figuren sind gleiche oder gleichwirkende Merkmale mit gleichen Bezugszeichen versehen. Dabei zeigen die Figuren eine mögliche Ausführungsform und die Montage eines Gelenkaufbaus. Von dieser ausgehend werden nachfolgend auch andere Ausführungsformen erläutert, die ebenfalls in den Schutzbereich der Ansprüche fallen. Insbesondere zeigt
Figur 1 einen Gelenkkörper mit einem einzuführenden Verriegelungselement,
Figur 2 einen Gelenkaufbau mit einer Gelenkaufnahme und einem Gelenkkörper,
Figur 3 den modularen Aufbau einer zweiten Gelenkkomponente und einer Gelenkaufnahme,
Figur 4 das Befestigen der Gelenkaufnahme an der zweiten Gelenkkomponente,
Figur 5 zwei in eine erste Gelenkkomponente einsetzbare Adapterelemente,
Figur 6 eine in eine zweite Gelenkkomponente eingesetzte Gelenkaufnahme und eine erste mit dieser zu montierenden Gelenkkomponente,
Figur 7 eine erste und eine zweite Gelenkkomponente, die für ein Einführen eines Gelenkkörpers und eines Verriegelungselements zueinander ausgerichtet sind, und
Figur 8 eine Ausführungsform eines Gelenkaufbaus, der eine erste und eine zweite Gelenkkomponente miteinander verbindet.

### AUSFÜHRLICHE BESCHREIBUNG BEVORZUGTER AUSFÜHRUNGSFORMEN

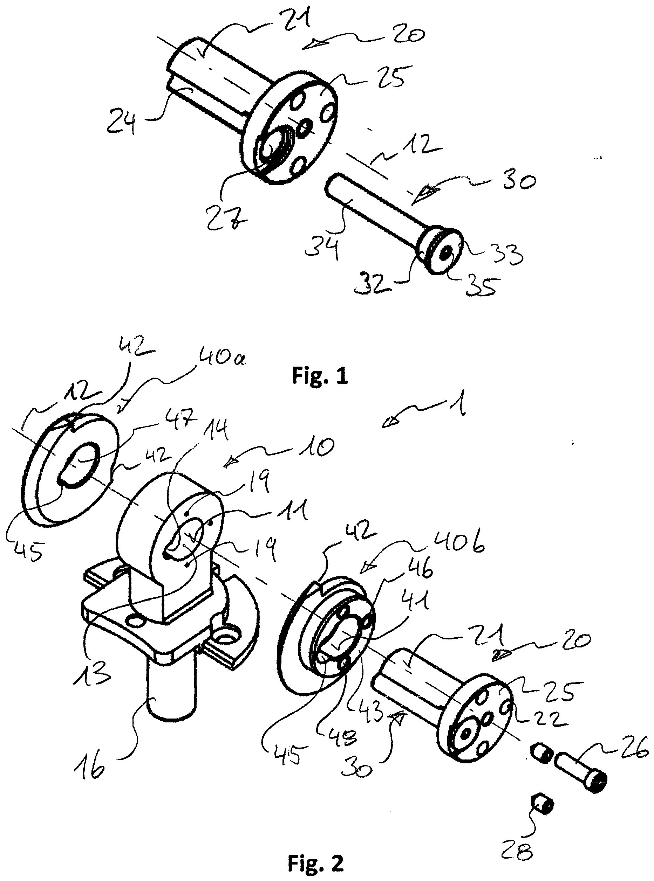

In Figur 1 ist eine mögliche Ausführungsform eines Gelenkkörpers 20 dargestellt, in den ein Verriegelungselement 30 einführbar ist. Der Gelenkkörper 20 weist eine Gelenkkörperfläche 21 auf, die zusammen mit einer Gelenkaufnahmefläche 11 ein Scharniergelenk ausbildet, das um eine Rotationsachse 12 schwenkbar ist. Dementsprechend ist die Gelenkkörperfläche zylindrisch mit einem kreisförmigen Querschnitt ausgebildet.

Die Gelenkkörperfläche 21 weist eine Körperverriegelungsstruktur 24 auf, die bei dem in Figur 1 gezeigten Ausführungsbeispiel als Aussparung vorgesehen ist, die sich in die Gelenkkörperfläche 21 erstreckt. Wie gezeigt, kann sich die Aussparung über die gesamte Breite der Gelenkkörperfläche 21 erstrecken. Allerdings ist es auch möglich, dass sich die Aussparung nur teilweise über die Breite der Gelenkkörperfläche 21 erstreckt.

Bei einer anderen Ausführung der Körperverriegelungsstruktur 24 kann diese an dem Gelenkkörper 20 auch innerhalb der Gelenkkörperfläche 21 oder an mindestens einer Stirnfläche des Gelenkkörpers 20 vorgesehen sein.

In der Figur 1 weist der Gelenkkörper 20 an einem Ende einen Sicherungsabschnitt 25 auf, der bei dem vorliegenden Ausführungsbeispiel als Flansch ausgeführt ist. Dieser stellt, wie oben bereits beschrieben, eine definierte Einführtiefe des Gelenkkörpers 20 in eine Gelenkaufnahme 10 (siehe Figur 2) bereit.

Das ebenfalls in Figur 1 veranschaulichte Verriegelungselement 30 ermöglicht eine indirekte Verriegelung der Körperverriegelungsstruktur 24 und einer Aufnahmeverriegelungsstruktur 14 der Gelenkaufnahme 10 (siehe Figur 2). Ebenso ist es möglich, eine direkte Verriegelung zwischen der Körperverriegelungsstruktur und der Aufnahmeverriegelungsstruktur vorzusehen. In so einem Fall sind die Körperverriegelungsstruktur und die Aufnahmeverriegelungsstruktur komplementär zueinander ausgeführt, sodass kein Verriegelungselement notwendig ist, um eine Verriegelung zwischen dem Gelenkkörper 20 und der Gelenkaufnahme 10 hervorzurufen.

Das Verriegelungselement 30 ist in der in Figur 1 gezeigten Ausführungsform parallel zu und in einem Abstand von der Rotationsachse 12 in die Aufnahme der Körperverriegelungsstruktur 24 einführbar. Die Aussparung der Körperverriegelungsstruktur 24 ist komplementär zu der Form des Verriegelungselement 30 ausgeführt. In der gezeigten beispielhaften Ausführungsform weist sie einen teilkreisförmigen Querschnitt auf.

Weiterhin ist das Verriegelungselement 30 von der Seite aus in die Körperverriegelungsstruktur 24 einführbar, auf welcher der Sicherungsabschnitt 25 des Gelenkkörpers 20 vorgesehen ist. Hierfür weist der als Flansch ausgebildete Sicherungsabschnitt 25 ein Durchgangsloch 27 auf. Das Durchgangsloch 27 ist vorzugsweise mit einem Gewinde ausgebildet, in das ein Gewindeabschnitt 32 des Verriegelungselements 30 eingreift, um das Verriegelungselement 30 an dem Gelenkkörper 20 zu sichern. Durch diese Sicherung wird ein unbeabsichtigtes Lösen der Verriegelung verhindert.

Der Gewindeabschnitt 32 weist vorzugsweise einen größeren Durchmesser auf als der mit der Körperverriegelungsstruktur 24 über einen Formschluss in Eingriff bringbare Verriegelungsabschnitt 34 des Verriegelungselements 30. Weiterhin weist das Verriegelungselement 30 an seinem in Einführrichtung hinteren Ende hinter dem Gewindeabschnitt 32 einen Kopf 33 auf, in dem für einen Eingriff eines Werkzeugs ein Werkzeugeingriffsabschnitt 35 vorgesehen ist, um den Gewindeabschnitt 32 in das mit einem Gewinde vorgesehene Durchgangsloch 27 des Gelenkkörpers 20 zu schrauben.

Die einen Gelenkaufbau 1 veranschaulichende Figur 2 zeigt das in Figur 1 beschriebene Verriegelungselement 30 im Eingriff mit der Körperverriegelungsstruktur 24 des Gelenkkörpers 20. Wie der Figur 2 zu entnehmen ist, ist der Gelenkkörper 20 in ein Durchgangsloch 13 entlang der Rotationsachse 12 des Gelenks einführbar. Zumindest ein Teil der Innenfläche des Durchgangslochs 13 bildet die Gelenkaufnahmefläche 11 aus, die im montierten unverriegelten Zustand relativ zu der Gelenkkörperfläche 21 des Gelenkkörpers 20 bewegbar ist.

Bei der in Figur 2 veranschaulichten Ausführungsform wird die Gelenkaufnahmefläche 11 durch eine Aussparung unterbrochen, welche die Aufnahmeverriegelungsstruktur 14 ausbildet. Diese Aussparung erstreckt sich über die gesamte Tiefe des Durchgangslochs 13. Wie bereits im Zusammenhang mit der Körperverriegelungsstruktur 24 beschrieben, ist allerdings ebenso eine Ausführung der Aussparung möglich, die sich nur über einen Teil der Tiefe des Durchgangslochs 13 erstreckt. Wie oben bereits ausgeführt, hat ein solches Anordnen der Verriegelungsstrukturen 14, 24 den Vorteil einer sehr stabilen Verriegelung mit wenig Spiel.

Wenn der Gelenkkörper 20 somit in die Gelenkaufnahme 10 eingeführt ist, ermöglicht das Verriegelungselement 30, welches im eingeführten Zustand sowohl mit der Körperverriegelungsstruktur 24 als auch mit der Aufnahmeverriegelungsstruktur 14 in Eingriff ist, eine Verriegelung des Scharniergelenks, die gegen eine Relativbewegung zwischen dem Gelenkkörper 20 und der Gelenkaufnahme 10 vorbeugt. Bei gelöstem bzw. entferntem Verriegelungselement 30 ist hingegen eine Relativbewegung zwischen dem Gelenkkörper 20 und der Gelenkaufnahme 10 möglich, sodass der Gelenkaufbau eine normale Funktion der Gelenkendoprothese ermöglicht.

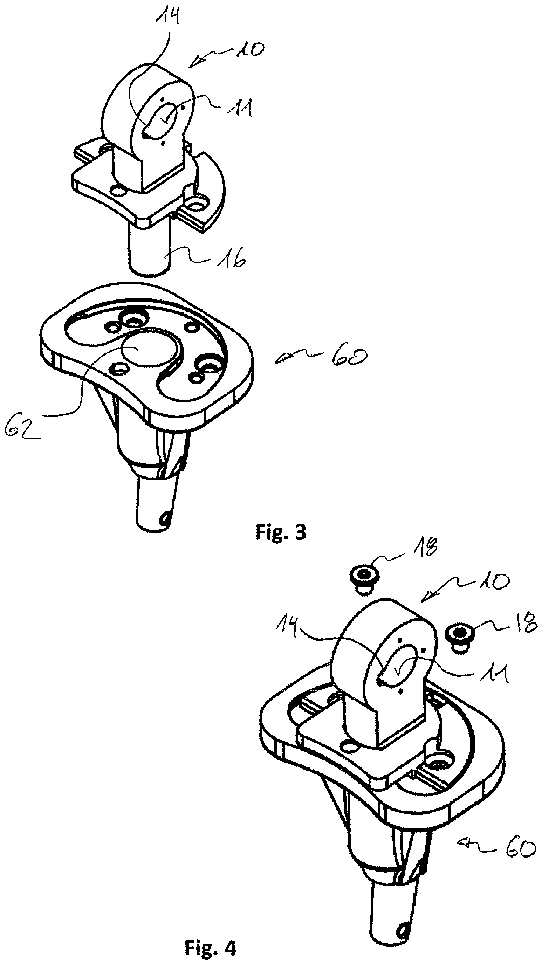

Wie weiter der Figur 2 zu entnehmen ist, weist die Gelenkaufnahme 10 einen Verbindungsabschnitt 16 auf, der, wie weiter unten noch näher beschrieben wird, für das Herstellen einer Verbindung mit einer zweiten Gelenkkomponente 60 dient (siehe Figuren 3 und 4). Bei der in Figur 2 gezeigten beispielhaften Ausführungsform der Gelenkaufnahme 10 handelt es sich um eine Gelenkaufnahme, die sich auf der tibialen Seite eines Kniegelenkersatzes befindet.

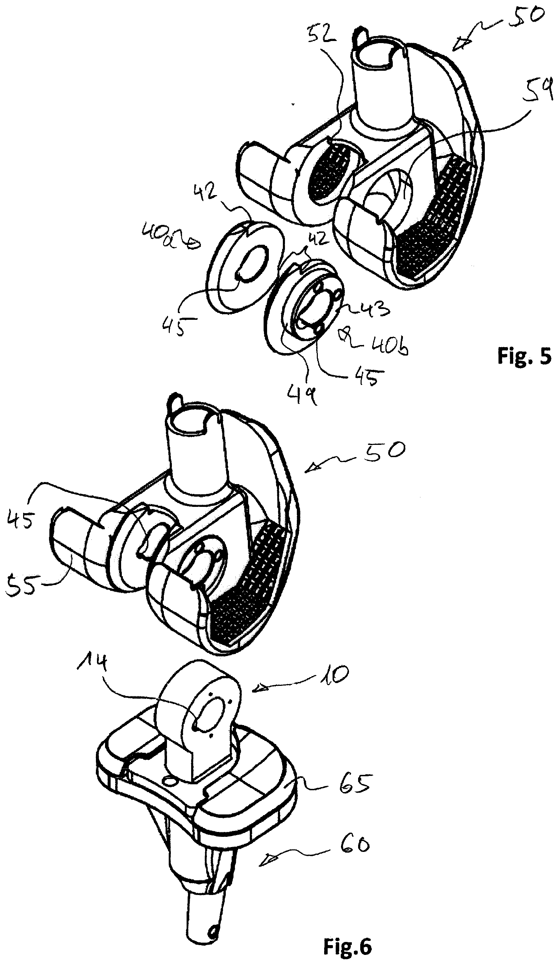

Weiterhin sind in Figur 2 zwei Adapterelemente 40a, 40b dargestellt, die beidseitig von dem Durchgangsloch 13 der Gelenkaufnahme 10 angeordnet sind. Wie weiter unten im Zusammenhang mit den Figuren 5 und 6 beschrieben wird, sind die Adapterelemente 40a, 40b seitlich zu der ersten Gelenkkomponente 50 angeordnet oder eingesetzt.

Die Adapterelemente 40a, 40b weisen Arretierungen 42 auf, die einer Drehung um die Rotationsachse 12 relativ zu der ersten Gelenkkomponente 50 vorbeugen. Alternativ zu den nachfolgend weiter im Detail beschriebenen Adapterelementen ist es auch möglich, eine erste Gelenkkomponente 50 vorzusehen, die ohne Adapterelement ein in diese intergriertes Durchgangsloch 47 mit Verriegelungselementaussparung 45 aufweist.

Das Adapterelement 40b weist auf der dem Sicherungsabschnitt 25 zugewandten Seite eine Anschlagfläche 43 auf, die bei eingeführtem Gelenkkörper 20 den Sicherungsabschnitt 25 berührt.

Wie bei der gezeigten Ausführungsform des Adapterelements 40b erkennbar, weist dieses ein Durchgangsloch 47 mit einer Verriegelungselementaussparung 45 auf. Die Verriegelungselementaussparung 45 ist dabei zumindest abschnittsweise so ausgeführt, dass sie einen Teil des Gewindeabschnitts 32 von dem Verriegelungselement 30 aufnimmt, jedoch bevorzugt nicht mit diesem in Gewindeeingriff geht.

Die Verriegelungselementaussparung 45 verengt sich vorzugsweise zu der der Anschlagflächen 43 gegenüberliegenden Seite des Adapterelements 40b hin, wie bei dem zweiten Adapterelement 40a in Figur 2 erkennbar, sodass danach lediglich der Verriegelungsabschnitt 34 des Verriegelungselements 30 einführbar ist. Es ist verständlich, dass das Adapterelement 40b mit einer Verriegelungselementaussparung 45 ausgeführt sein kann, die durchgehend nur für die Aufnahme des Verriegelungsabschnitts 34 vorgesehen ist.

Auch wenn bevorzugt bei beiden Adapterelementen 40a, 40b eine Verriegelungselementaussparung 45 für den Verriegelungsabschnitt 34 vorgesehen ist, so sind dennoch Ausführungsformen denkbar, bei denen nur das auf der Seite des Sicherungsabschnitts 25 befindliche Adapterelement 40b eine Verriegelungselementaussparung 45 aufweist. Jedoch ist bei einer Aufnahme des Verriegelungsabschnitts 34 in beiden Adapterelementen 40a, 40b eine stabilere Verriegelung möglich, da sich das Verriegelungselement 30 beidseitig von der Gelenkaufnahme 10 abstützen kann.

Das Adapterelement 40b weist zudem bei der in Figur 2 gezeigten Ausführungsform in der Anschlagfläche 43 eine Aufnahme 46 für ein Befestigungselement 26 auf. Diese Aufnahme 46 ist bevorzugt als Gewindeloch ausgebildet. In die Aufnahme 46 ist das Befestigungselement 26 einführbar, das über ein Durchgangsloch 22 in dem Sicherungsabschnitt 25 des Gelenkkörpers 20 den Gelenkkörper 20 an dem Adapterelement 40b sichert. Hierdurch kann gewährleistet werden, dass im unverriegelten Zustand des Gelenkaufbaus 1 eine Relativbewegung nur zwischen der Gelenkaufnahmefläche 11 und der Gelenkkörperfläche 21 möglich ist.

Wie oben beschrieben, kann hierauf bei alternativen Ausführungsformen verzichtet werden. Beispielsweise ist es möglich, den Gelenkkörper an beiden Enden über als Flansche ausgebildete Sicherungsabschnitte 25 in dem Durchgangsloch 13 der Gelenkaufnahme 10 und dem Durchgangsloch 47 der Adapterelemente 40 zu sichern.

Wie weiterhin in der Figur 2 zu erkennen, ist das Verriegelungselement 30 nur dann in das Durchgangsloch 27 des Gelenkkörpers 20, die Verriegelungselementaussparung 45 in dem Adapterelement 40b, die Aufnahmeverriegelungsstruktur 14 und die Verriegelungselementaussparung 45 des Adapterelements 40a einführbar, wenn diese eine Verriegelungsposition einnehmen, in der sie zueinander fluchten.

Für den Fall, dass der Gelenkkörper 20 anstatt über das Adapterelement 40 direkt mit einer ersten Gelenkkomponente 50 verbunden ist, müssen dementsprechend zumindest die Aufnahmeverriegelungsstruktur 14 und die Körperverriegelungsstruktur 24 miteinander fluchten und bei Verwendung eines Verriegelungselements 30 entsprechend die der Verriegelungselementaussparung 45 entsprechende Aussparung in der ersten Gelenkkomponente 50.

Der Winkel zwischen der ersten Gelenkkomponente 50 und der zweiten Gelenkkomponente 60, den der Gelenkaufbau 1 im verriegelten Zustand bereitstellt, ist je nach Ausführungsform durch eine entsprechende relative Anordnung der Aufnahmeverriegelungsstruktur 14, der Körperverriegelungsstruktur 24, der Verriegelungselementaussparung 45 und/oder der Arretierungen 42 um die Rotationsachse 12 zueinander einstellbar.

Bei der dargestellten Ausführungsform ist eine Einstellung dieses Winkels am einfachsten durch eine entsprechende Anordnung der Arretierungen 42 an den Adapterelementen 40a und 40b möglich. Folglich können durch eine entsprechende Platzierung der Arretierungen 42 an den Adapterelementen 40a und 40b unterschiedliche Adapterelemente für unterschiedliche Verriegelungswinkel vorgesehen sein.

Bei einer anderen oder ergänzenden Ausführung ist es möglich, wie oben bereits beschrieben, mehrere Aufnahmeverriegelungsstrukturen 14 und/oder Körperverriegelungsstrukturen 24 vorzusehen, um für die Verriegelung mehrere Verriegelungswinkel für die Gelenkendoprothese 90 zur Verfügung zu haben.

Besonders bevorzugt werden zwei oder drei Aufnahmeverriegelungsstrukturen 14 vorgesehen, die, wie beschrieben, noch bevorzugter als Aussparung in der Gelenkaufnahme 11 ausgebildet sind. Beispielsweise kann ein Operateur je nach Patient eine erste für eine Extension der Gliedmaße angeordnete Aufnahmeverriegelungsstruktur 14 oder eine zweite für eine Beugung der Gliedmaße angeordnete Aufnahmeverriegelungsstruktur 14 auswählen. Es ist auch denkbar eine Verriegelungsposition zwischen einer Extension und einer Beugung des Gelenks zu wählen, die beispielsweise für ein Gehen mit einer Gehhilfe von Vorteil sein kann.

Um intraoperativ durch eine Relativbewegung zwischen der Gelenkaufnahmefläche 11 und der Gelenkkörperfläche 21 die Verriegelungsposition leichter zu finden, kann weiterhin ein Zentriermittel 28 vorgesehen sein, dass bei Erreichen der Verriegelungsposition spürbar in eine Zentrieraussparung 19 der Gelenkaufnahme 10 eingreift. Bei der in Figur 2 gezeigten Ausführungsform wird als Zentriermittel 28 ein Zentrierstift vorgesehen, der durch ein Zentrierdurchgangsloch 29 in dem Sicherungsabschnitt 25 des Gelenkkörpers 20 in einer Zentrieraussparung 19 einrasten kann, was einem Operateur signalisiert, dass die Verriegelungsposition erreicht worden ist und somit das Verriegelungselement 30 eingeführt werden kann.

Im Folgenden wird nunmehr die Montage des Gelenkaufbaus 1 näher anhand eines Kniegelenkersatzes beschrieben, ist aber ebenso an anderen Gelenken durchführbar, wie zum Beispiel einem Ellenbogengelenk.

Die in Figur 3 dargestellte zweite Gelenkkomponente 60 ist eine Tibiakomponente, die bei einer Nachrüstung des Gelenkaufbaus 1 im Falle einer Nachrüstung bereits in dem Knie eines Patienten implantiert sein kann oder als neues Implantat zu implantieren ist. Für eine Montage des Gelenkaufbaus wird in einem Schritt nach Öffnung des Kniegelenks der Verbindungsabschnitt der Gelenkaufnahme 10 in einen korrespondierenden Verbindungsabschnitt 62 der zweiten Gelenkkomponente 60 eingesetzt. Wie in den Figuren 3 und 4 zu erkennen, erfolgt die Montage der Gelenkaufnahme 10 mit der zweiten Gelenkkomponente 60 über einen Formschluss und anschließender Fixierung mit Befestigungselementen 18. Alternativ sind auch andere Befestigungsmöglichkeiten der zweiten Gelenkkomponente 60 mit dem Gelenkaufbau 10 denkbar, wie zum Beispiel eine Konusverbindung.

Wie für die zweite Gelenkkomponente 60 beschrieben, kann auch die in den Figuren 5 und 6 gezeigte erste Gelenkkomponente 50 bereits implantiert sein, sodass ein zuvor vorhandener Gelenkaufbau durch den Gelenkaufbau 10 ausgetauscht wird, oder neu zusammen mit dem Gelenkaufbau 10 implantiert werden.

Bei der in der beispielhaften Ausführungsform in Figur 5 gezeigten ersten Gelenkkomponente 50 handelt es sich um eine Femurkomponente eines Kniegelenks. Zudem werden im Rahmen des Gelenkaufbaus 10 Adapterelemente 40a, 40b eingesetzt. Folglich handelt es sich bei dem Gelenkaufbau 10, wie oben beschrieben, um ein modulares Systems, das den Gelenkaufbau einer gewöhnlichen Gelenkendoprothese mit dem verriegelten Gelenkaufbau 1 der vorliegenden Erfindung ersetzen kann.

Im vorliegenden Fall werden die Adapterelemente 40a, 40b jeweils über einen Einsetzvorsprung 49 in entsprechende Durchgangslöcher 59 der ersten Gelenkkomponente 50 eingesetzt. Bei Verwendung der ersten Gelenkkomponente 50 mit einem gewöhnlichen nicht verriegelbaren Gelenkaufbau können diese Durchgangslöcher 59 für eine Gelenkachse vorgesehen sein. Die Arretierungen 42 der Adapterelemente 40a, 40b werden beim Einsetzen der Adapterelemente 40a, 40b mit entsprechenden Arretierungen 52 der ersten Gelenkkomponente 50 in Eingriff gebracht. Wie in Figur 5 zu erkennen ist, weist jedes Adapterelement 40 zwei Arretierungen 42 auf, die mit zwei Arretierungen 52 der ersten Gelenkkomponente zusammenwirken, um das jeweilige Adapterelement 40 in dem entsprechenden Durchgangsloch 59 in Rotationsrichtung zu fixieren.

Vorzugsweise kann über die Wahl der Adapterelemente 40 die Verriegelungsposition und damit der Winkel zwischen der ersten Gelenkkomponente 50 und der zweiten Gelenkkomponente festgelegt werden, indem die Arretierungen entsprechend am Umfang der Adapterelemente vorgesehen werden.

Wie den Figuren 5 und 6 anhand der durch die Arretierungen 42 und 52 positionierte Verriegelungselementaussparung 45 und der Aufnahmeverriegelungsstruktur 14 erkennbar, befindet sich der beispielhafte Kniegelenkersatz im verriegelten Zustand in Extension. Wie oben bereits erläutert, kann es jedoch von Vorteil sein, einen anderen Winkel zu wählen, wie zum Beispiel einen Winkel, mit dem der Patient eine Sitzstellung einnehmen kann.

Figur 6 zeigt die mit den Adapterelementen 40a, 40b vormontierte erste Gelenkkomponente 50 und die mit der Gelenkaufnahme 10 vormontierte zweite Gelenkkomponente 60 vor deren Positionierung zueinander. Wie in Figur 6 zu erkennen, kann die erste Gelenkkomponente 50 neben dem durch den Gelenkaufbau 1 ausgebildeten Gelenk eine weitere Gelenkfläche 55 aufweisen, die im montierten Zustand des Gelenks mit einer an der zweiten Gelenkkomponente montierten Gelenkfläche 65 zusammenwirkt.

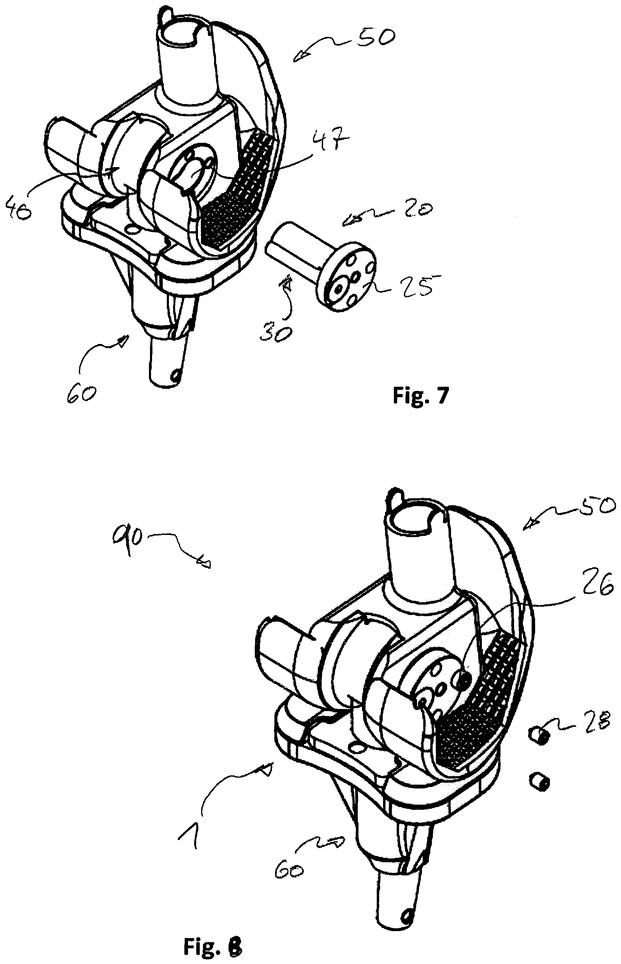

In Figur 7 sind die erste Gelenkkomponente 50 und die zweite Gelenkkomponente 60 so zueinander angeordnet, dass das Durchgangsloch 47 der Adapterelemente 40a und 40b und das Durchgangsloch 13 der Gelenkaufnahme 10 zueinander fluchten. Folglich kann in diesem Zustand der Gelenkkörper 20 über das Durchgangsloch 47 des ersten Adapterelements 40b, das Durchgangsloch 13 der Gelenkaufnahme 10 und das Durchgangsloch 47 des zweiten Adapterelements 40a eingeführt werden.

In diesem Zusammenhang ist anzumerken, dass wenn es sich bei der ersten Gelenkkomponente 50 um eine bereits implantierte Gelenkkomponente handelt, sich vor den Durchgangslöchern 59 der ersten Gelenkkomponente 50 Knochengewebe befinden kann. Dementsprechend muss intraoperativ zunächst ein Durchgang zu zumindest einem der Durchgangslöcher 59 geschaffen werden. Dies erfolgt vorzugsweise mit einer Hohlfräse, mit der ein Knochenzylinder mit einem Durchmesser seitlich entlang der Rotationsachse 12 des Gelenks entnommen wird, sodass ein freier Zugang zu dem mindestens einen Durchgangsloch 59 der ersten Gelenkkomponente 50 entsteht. Nach der Montage kann der entnommene Knochenzylinder wieder eingesetzt werden, um den erzeugten Zugang zu dem jeweiligen Durchgangsloch 59 wieder zu verschließen.

Bei der in den Figuren gezeigten Ausführungsform erfolgt die Sicherung des Gelenkkörpers über einen einzigen als Flansch ausgeführten Sicherungsabschnitt 25. Folglich reicht ein Zugang zu einem der Durchgangslöcher 59, um den Gelenkersatz mit dem Gelenkaufbau 1 nachzurüsten. Dies hat den Vorteil, dass das Knochengewebe und damit der Patient geschont werden.

Weiterhin ist der Figur 7 zu entnehmen, dass der Gelenkkörper 20 mit bereits einem in Eingriff befindlichen Verriegelungselement 30 eingesetzt werden kann. Es ist verständlich, dass dies nicht notwendig ist, sondern stattdessen das Verriegelungselement 30 erst dann eingesetzt wird, wenn eine Verriegelung des Gelenks gewünscht ist oder auch für eine Verriegelung erst der Gelenkkörper 20 und dann das Verriegelungselement 30 eingesetzt wird.

Wenn, wie in Figur 7 gezeigt, der Gelenkkörper 20 und das Verriegelungselement 30 auf diese Weise eingesetzt werden, müssen bei der gezeigten Ausführungsform dementsprechend neben den vorgenannten Durchgangslöchern 47 und 13 auch die Verriegelungselementaussparung 45 von zumindest einem der Adapterelemente 40a, 40b sowie die als Aussparung ausgeführte Aufnahmeverriegelungsstruktur 14 fluchten, d. h. sich in der Verriegelungsposition befinden.

Figur 8 zeigt schließlich den Gelenkaufbau 1 in einem mit der ersten Gelenkkomponente 50 und der zweiten Gelenkkomponente 60 montierten Zustand. Wie zu erkennen ist, ist der Gelenkkörper 20 über den Sicherungsabschnitt 25 und das Befestigungselement 26 an der ersten Gelenkkomponente 50 gesichert. Folglich ist bei der gezeigten Ausführungsform in dem Gelenkaufbau 1 lediglich eine Relativbewegung zwischen der Gelenkaufnahmefläche 11 und der Gelenkkörperfläche 21 möglich.

Dies hat unter anderem den Vorteil, dass das durch den Gelenkaufbau 1 ausgebildete Gelenk von der Knochenseite her abgeschlossen ist, sodass einem Eindringen von Fremdpartikeln in das Gelenk vorgebeugt wird. Als Ergänzung ist es möglich, statt eines Durchgangsloch 47 bei dem Adapterelement 40a, das dem Adapterelement 40b gegenüberliegt, an dem der Sicherungsabschnitt 25 des Gelenkkörpers 20 anliegt, ein Sackloch vorzusehen oder auch eine Kappe, die dasDurchgangsloch 47 des Adapterelements 40a wieder bedeckt. Damit wird auch einem Eindringen von Fremdpartikeln von der anderen Seite des Gelenkaufbaus 1 aus vorgebeugt.

Weiterhin wird in der Figur 8 dargestellt, dass die Zentriermittel entnommen werden können. Alternativ oder ergänzend ist es möglich, die Zentriermittel nicht wie zuvor beschrieben als Hilfsmittel einzusetzen, um die Verriegelungsposition zu finden, sondern diese stattdessen als zusätzliche Sicherungselemente gegen ein Verdrehen des Gelenkkörpers 20 relativ zu der ersten Gelenkkomponente 50 einzusetzen.

### BEZUGSZEICHEN

- 1: Gelenkaufbau
- 10: Gelenkaufnahme
- 11: Gelenkaufnahmefläche
- 12: Rotationsmittelpunkt
- 13: Durchgangsloch
- 14: Aufnahmeverriegelungsstruktur
- 16: Verbindungsabschnitt
- 18: Befestigungselement
- 19: Zentrieraussparung
- 20: Gelenkkörper
- 21: Gelenkkörperfläche
- 22: Durchgangsloch für das Befestigungselement 26
- 24: Körperverriegelungsstruktur
- 25: Sicherungsabschnitt
- 26: Befestigungselement
- 27: Durchgangsloch
- 28: Zentriermittel
- 29: Zentrierdurchgangsloch
- 30: Verriegelungselement
- 32: Gewindeabschnitt des Verriegelungselements
- 33: Kopf des Verriegelungselements
- 34: Verriegelungsabschnitt
- 40a,b: Adapterelement
- 41: Stützfläche für Gelenkkörper
- 42: Arretierung
- 43: Anschlagfläche
- 44: Stützfläche für Verriegelungselement
- 45: Verriegelungselementaussparung
- 46: Aufnahme für das Befestigungselement 26
- 47: Durchgangsloch zur Aufnahme des Gelenkkörpers
- 48: Aufnahme für das Zentriermittel 28
- 49: Einsetzvorsprung
- 50: erste Gelenkkomponente
- 52: Arretierung
- 55: Gelenkfläche der ersten Gelenkkomponente
- 59: Durchgangsloch der ersten Gelenkkomponente
- 60: zweite Gelenkkomponente
- 62: Verbindungsabschnitt
- 65: Gelenkfläche der zweiten Gelenkkomponente
- 70: dritte Gelenkkomponente
- 80: vierte Gelenkkomponente
- 90: Gelenkendoprothese

## Patentansprüche

1. Gelenkaufbau (1) für eine ein Scharniergelenk ausbildende Gelenkendoprothese (90), der aufweist:
- eine Gelenkaufnahme (10), die eine Gelenkaufnahmefläche (11) und eine Aufnahmeverriegelungsstruktur (14) aufweist,
- einen Gelenkkörper (20), der in der Gelenkaufnahme (10) aufnehmbar ist und eine Gelenkkörperfläche (21) und eine Körperverriegelungsstruktur (24) aufweist,
wobei die Gelenkaufnahme (10) und der Gelenkkörper (20) relativ zueinander um eine Rotationsachse (12) schwenkbar sind,
die Gelenkaufnahmefläche (11) und die Gelenkkörperfläche (21) relativ zueinander in eine Stellung bewegbar sind, in der die Aufnahmeverriegelungsstruktur (14) und die Körperverriegelungsstruktur (24) eine Verriegelungsposition einnehmen, in der sie für einen verriegelten Zustand zwischen der Gelenkaufnahme (10) und dem Gelenkkörper (20) in Eingriff bringbar sind,
die Aufnahmeverriegelungsstruktur (14) und die Körperverriegelungsstruktur (24) mit einem Verriegelungselement (30) in Eingriff bringbar sind, **dadurch gekennzeichnet, dass**
das Verriegelungselement (30) im Wesentlichen parallel zu der Rotationsachse (12) einführbar ist.

2. Gelenkaufbau nach Anspruch 1, bei dem die Aufnahmeverriegelungsstruktur (14) an die Gelenkaufnahmefläche (11) und die Körperverriegelungsstruktur (24) an die Gelenkkörperfläche (21) angrenzt.

3. Gelenkaufbau nach Anspruch 1 oder 2, bei dem die Aufnahmeverriegelungsstruktur (14) als Aussparung in der Gelenkaufnahmefläche (11) und/oder die Körperverriegelungsstruktur (24) als Aussparung in der Gelenkkörperfläche (21) ausgebildet ist.

4. Gelenkaufbau nach einem der vorstehenden Ansprüche, bei dem das Verriegelungselement (30) in der Verriegelungsposition in die Aufnahmeverriegelungsstruktur (14) und die Körperverriegelungsstruktur (24) einführbar ist.

5. Gelenkaufbau nach einem der vorstehenden Ansprüche, bei dem der Gelenkkörper (20) einen Sicherungsabschnitt (25) für eine Sicherung des Gelenkkörpers (20) an einer ersten Gelenkkomponente (50) aufweist, wobei der Sicherungsabschnitt (25) vorzugsweise als Flansch an einem Ende des Gelenkkörpers (20) ausgebildet ist.

6. Gelenkaufbau nach Anspruch 5, bei dem der Sicherungsabschnitt (25) mit einem Befestigungselement (26) an der ersten Gelenkkomponente (50) befestigt ist.

7. Gelenkaufbau nach Anspruch 5 oder 6, bei dem mindestens ein Adapterelement (40) zwischen dem Gelenkkörper (20) und der ersten Gelenkkomponente (50) vorgesehen ist, das eine Stützfläche (41) zum Unterstützen der Gelenkkörperfläche (21) aufweist.

8. Gelenkaufbau nach Anspruch 7, bei dem das Adapterelement (40) eine Arretierung (42) aufweist, die einer Drehung zwischen dem Adapterelement (40) und der ersten Gelenkkomponente (50) vorbeugt.

9. Gelenkaufbau nach einem der Ansprüche 4 bis 8, bei dem das Verriegelungselement (30) vorzugweise über einen Gewindeabschnitt (32) relativ zu dem Gelenkkörper (20) fixierbar ist.

10. Gelenkaufbau nach einem der vorstehenden Ansprüche, bei dem die Gelenkaufnahme (10) einen Verbindungsabschnitt (16) für eine Verbindung mit einer zweiten Gelenkkomponente (60) aufweist.

11. Gelenkaufbau nach einem der vorstehenden Ansprüche, der mindestens eine weitere Aufnahmeverriegelungsstruktur (14) und/oder eine weitere Körperverriegelungsstruktur (24) aufweist, die angeordnet ist, um mindestens eine weitere Verriegelungsposition für eine Verriegelung des Gelenkaufbaus bereitzustellen.

12. Verfahren zur Verriegelung eines Gelenkaufbaus (1) für eine ein Scharniergelenk ausbildende Gelenkendoprothese (90), wobei das Verfahren die Schritte umfasst:
- Einführen eines Gelenkkörpers (20), der eine Gelenkkörperfläche (21) und eine Körperverriegelungsstruktur (24) aufweist, in eine Gelenkaufnahme (10), die eine Gelenkaufnahmefläche (11) und eine Aufnahmeverriegelungsstruktur (14) aufweist, wobei die Gelenkaufnahme (10) und der Gelenkkörper (20) relativ zueinander um eine Rotationsachse (12) schwenkbar sind;
- Bewegen der Gelenkkörperfläche (21) und der Gelenkaufnahmefläche (11) relativ zueinander, sodass die Körperverriegelungsstruktur (24) und die Aufnahmeverriegelungsstruktur (14) eine Verriegelungsposition einnehmen;
- Verriegeln des Gelenkkörpers (20) und der Gelenkaufnahme (10) in der Verriegelungsposition durch in Eingriff bringen der Körperverriegelungsstruktur (24) und der Aufnahmeverriegelungsstruktur (14), wobei die Verriegelung zwischen der Körperverriegelungsstruktur (24) und der Aufnahmeverriegelungsstruktur (14) mit einem Verriegelungselement (30) erfolgt, **dadurch gekennzeichnet, dass**
das Verriegelungselement (30) im Wesentlichen parallel zu der Rotationsachse (12) einführbar ist.

13. Verfahren zur Montage eines Gelenkaufbaus nach Anspruch 12, bei dem der Gelenkkörper (20) über ein Adapterelement (40) mit einer ersten Gelenkkomponente (50) verbunden wird, wobei das Adapterelement (40) vorzugsweise eine Stützfläche (44), die mit der Gelenkaufnahmefläche (11) der Gelenkaufnahme (10) in Kontakt gebracht wird, und eine Arretierung (42), die einer relativen Drehung zwischen dem Adapterelement (40) und der ersten Gelenkkomponente (50) vorbeugt, aufweist.

14. Verfahren zur Montage eines Gelenkaufbaus nach einem der Ansprüche 12 bis 13, bei dem die Gelenkaufnahme (10) an einer zweiten Gelenkkomponente (60) befestigt wird.

## Claims

1. Joint configuration (1) for a joint endoprosthesis (90) forming a hinge joint, having:
- a joint receptacle (10), which has a joint receptacle surface (11) and a receptacle locking structure (14),
- a joint body (20), which is receivable in the joint receptacle (10) and has a joint body surface (21) and a body locking structure (24),
wherein the joint receptacle (10) and the joint body (20) are pivotable relative to each other about a rotation axis (12),
the joint receptacle surface (11) and the joint body surface (21) are movable relative to each other into a position in which the receptacle locking structure (14) and the body locking structure (24) assume a locking position, in which they can be brought into engagement for a locked state between the joint receptacle (10) and the joint body (20),
the receptacle locking structure (14) and the body locking structure (24) can be brought into engagement with a locking element (30), **characterized in that**
the locking element (30) is insertable substantially parallel to the rotation axis (12).

2. Joint configuration according to Claim 1, in which the receptacle locking structure (14) adjoins the joint receptacle surface (11), and the body locking structure (24) adjoins the joint body surface (21).

3. Joint configuration according to Claim 1 or 2, in which the receptacle locking structure (14) is formed as a recess in the joint receptacle surface (11), and/or the body locking structure (24) is formed as a recess in the joint body surface (21).

4. Joint configuration according to one of the preceding claims, in which the locking element (30), in the locking position, is insertable into the receptacle locking structure (14) and the body locking structure (24) .

5. Joint configuration according to one of the preceding claims, in which the joint body (20) has a securing portion (25) for securing the joint body (20) to a first joint component (50), wherein the securing portion (25) is preferably formed as a flange at one end of the joint body (20).

6. Joint configuration according to Claim 5, in which the securing portion (25) is fastened to the first joint component (50) with a fastening element (26).

7. Joint configuration according to Claim 5 or 6, in which at least one adapter element (40) is provided between the joint body (20) and the first joint component (50), which adapter element (40) has a support surface (41) for supporting the joint body surface (21).

8. Joint configuration according to Claim 7, in which the adapter element (40) has a catch (42), which prevents a rotation between the adapter element (40) and the first joint component (50).

9. Joint configuration according to one of Claims 4 to 8, in which the locking element (30) is fixable relative to the joint body (20), preferably via a threaded portion (32).

10. Joint configuration according to one of the preceding claims, in which the joint receptacle (10) has a connecting portion (16) for a connection to a second joint component (60).

11. Joint configuration according to one of the preceding claims, which has at least one further receptacle locking structure (14) and/or one further body locking structure (24), which is arranged so as to provide at least one further locking position for locking of the joint configuration.

12. Method for locking a joint configuration (1) for a joint endoprosthesis (90) forming a hinge joint, wherein the method comprises the following steps:
- inserting a joint body (20), which has a joint body surface (21) and a body locking structure (24), into a joint receptacle (10), which has a joint receptacle surface (11) and a receptacle locking structure (14), wherein the joint receptacle (10) and the joint body (20) are pivotable relative to each other about a rotation axis (12);
- moving the joint body surface (21) and the joint receptacle surface (11) relative to each other, such that the body locking structure (24) and the receptacle locking structure (14) assume a locking position;
- locking the joint body (20) and the joint receptacle (10) in the locking position by bringing the body locking structure (24) and the receptacle locking structure (14) into engagement, wherein the locking between the body locking structure (24) and the receptacle locking structure (14) is effected using a locking element (30), **characterized in that**
the locking element (30) is insertable substantially parallel to the rotation axis (12).

13. Method for assembling a joint configuration according to Claim 12, in which method the joint body (20) is connected via an adapter element (40) to a first joint component (50), wherein the adapter element (40) preferably has a support surface (44), which is brought into contact with the joint receptacle surface (11) of the joint receptacle (10), and a catch (42), which prevents a relative rotation between the adapter element (40) and the first joint component (50).

14. Method for assembling a joint configuration according to either of Claims 12 and 13, in which method the joint receptacle (10) is fastened to a second joint component (60).

## Revendications

1. Construction d'articulation (1) pour une endoprothèse d'articulation (90) qui forme une articulation à charnière, laquelle comprend :
- un logement d'articulation (10), qui possède une surface d'accueil d'articulation (11) et une structure de verrouillage de logement (14),
- un corps d'articulation (20), qui peut être accueilli dans le logement d'articulation (10) et qui possède une surface de corps d'articulation (21) et une structure de verrouillage de corps (24),
le logement d'articulation (10) et le corps d'articulation (20) pouvant pivoter l'un par rapport à l'autre autour d'un axe de rotation (12),
la surface d'accueil d'articulation (11) et la surface de corps d'articulation (21) pouvant être déplacées l'une par rapport à l'autre dans une position dans laquelle la structure de verrouillage de logement (14) et la structure de verrouillage de corps (24) adoptent une position de verrouillage dans laquelle ils peuvent être amenés en prise pour un état verrouillé entre le logement d'articulation (10) et le corps d'articulation (20),
la structure de verrouillage de logement (14) et la structure de verrouillage de corps (24) pouvant être amenées en prise avec un élément de verrouillage (30), **caractérisée en ce que**
l'élément de verrouillage (30) peut être introduit sensiblement en parallèle à l'axe de rotation (12).

2. Construction d'articulation selon la revendication 1, dans laquelle la structure de verrouillage de logement (14) est adjacente de la surface d'accueil d'articulation (11) et la structure de verrouillage de corps (24) est adjacente de la surface de corps d'articulation (21).

3. Construction d'articulation selon la revendication 1 ou 2, dans laquelle la structure de verrouillage de logement (14) est réalisée sous la forme d'un évidement dans la surface d'accueil d'articulation (11) et/ou la structure de verrouillage de corps (24) sous la forme d'un évidement dans la surface de corps d'articulation (21) .

4. Construction d'articulation selon l'une des revendications précédentes, dans laquelle l'élément de verrouillage (30) en position de verrouillage peut être introduit dans la structure de verrouillage de logement (14) et la structure de verrouillage de corps (24).

5. Construction d'articulation selon l'une des revendications précédentes, dans laquelle le corps d'articulation (20) possède une portion de blocage (25) pour un blocage du corps d'articulation (20) au niveau d'un premier composant d'articulation (50), la portion de blocage (25) étant de préférence réalisée sous la forme d'une bride à une extrémité du corps d'articulation (20).

6. Construction d'articulation selon la revendication 5, dans laquelle la portion de blocage (25) est fixée au premier composant d'articulation (50) avec un élément de fixation (26).

7. Construction d'articulation selon la revendication 5 ou 6, dans laquelle un élément adaptateur (40) se trouve entre le corps d'articulation (20) et le premier composant d'articulation (50), lequel possède une surface d'appui (41) destinée à soutenir la surface de corps d'articulation (21).

8. Construction d'articulation selon la revendication 7, dans laquelle l'élément adaptateur (40) possède un dispositif d'arrêt (42) qui prévient une rotation entre l'élément adaptateur (40) et le premier composant d'articulation (50).

9. Construction d'articulation selon l'une des revendications 4 à 8, dans laquelle l'élément de verrouillage (30) peut être immobilisé par rapport au corps d'articulation (20) de préférence par le biais d'une portion filetée (32).

10. Construction d'articulation selon l'une des revendications précédentes, dans laquelle le logement d'articulation (10) possède une portion de liaison (16) pour une liaison avec un deuxième composant d'articulation (60).

11. Construction d'articulation selon l'une des revendications précédentes, laquelle possède au moins une structure de verrouillage de logement (14) supplémentaire et/ou une structure de verrouillage de corps (24) supplémentaire, qui est disposée de manière à fournir au moins une position de verrouillage supplémentaire pour un verrouillage de la construction d'articulation.

12. Procédé pour verrouiller une construction d'articulation (1) pour une endoprothèse d'articulation (90) qui forme une articulation à charnière, le procédé comprenant les étapes suivantes :
- introduction d'un corps d'articulation (20), qui possède une surface de corps d'articulation (21) et une structure de verrouillage de corps (24), dans un logement d'articulation (10), qui possède une surface d'accueil d'articulation (11) et une structure de verrouillage de logement (14), le logement d'articulation (10) et le corps d'articulation (20) pouvant pivoter l'un par rapport à l'autre autour d'un axe de rotation (12) ;
- déplacement de la surface de corps d'articulation (21) et de la surface d'accueil d'articulation (11) l'une par rapport à l'autre, de sorte que la structure de verrouillage de corps (24) et la structure de verrouillage de logement (14) adoptent une position de verrouillage ;
- verrouillage du corps d'articulation (20) et du logement d'articulation (10) dans la position de verrouillage par la mise en prise de la structure de verrouillage de corps (24) et de la structure de verrouillage de logement (14), le verrouillage entre la structure de verrouillage de corps (24) et la structure de verrouillage de logement (14) s'effectuant avec un élément de verrouillage (30), **caractérisé en ce que** l'élément de verrouillage (30) peut être introduit sensiblement en parallèle à l'axe de rotation (12).

13. Procédé de montage d'une construction d'articulation selon la revendication 12, dans lequel le corps d'articulation (20) est relié à un premier composant d'articulation (50) par le biais d'un élément adaptateur (40), l'élément adaptateur (40) possédant de préférence une surface d'appui (44) qui est mise en contact avec la surface d'accueil d'articulation (11) du logement d'articulation (10), et un dispositif d'arrêt (42) qui prévient une rotation relative entre l'élément adaptateur (40) et le premier composant d'articulation (50).

14. Procédé de montage d'une construction d'articulation selon l'une des revendications 12 à 13, dans lequel le logement d'articulation (10) est fixé à un deuxième composant d'articulation (60).
